(19) Europäisches Patentamt
European Patent Office
Office européen des brevets

(11) EP 2 344 650 B1

(12) **EUROPEAN PATENT SPECIFICATION**

(45) Date of publication and mention of the grant of the patent:
**23.04.2014 Bulletin 2014/17**

(21) Application number: **09743982.2**

(22) Date of filing: **30.09.2009**

(51) Int Cl.:
***C12P 7/00*** *(2006.01)*

(86) International application number:
**PCT/US2009/058997**

(87) International publication number:
**WO 2010/039812 (08.04.2010 Gazette 2010/14)**

# (54) IMPROVEMENT OF ENZYMATIC HYDROLYSIS OF PRE-TREATED LIGNOCELLULOSE-CONTAINING MATERIAL WITH DISTILLERS DRIED GRAINS

VERBESSERUNG DER ENZYMATISCHEN HYDROLYSE VON VORBEHANDELTEM LIGNOZELLUOSE-ENTHALTENDEN MATERIAL MIT TROCKENSCHLEMPE

AMÉLIORATION D'HYDROLYSE ENZYMATIQUE DE MATÉRIAU PRÉTRAITÉ CONTENANT DE LA LIGNOCELLULOSE AVEC DE LA DRÈCHE SÈCHE DE DISTILLERIE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **30.09.2008 US 101450 P**

(43) Date of publication of application:
**20.07.2011 Bulletin 2011/29**

(73) Proprietor: **Novozymes North America, Inc.**
**Franklinton, NC 27525 (US)**

(72) Inventors:
- **LI, Xin**
**Raleigh**
**North Carolina 27614 (US)**
- **HAAGENSEN, Frank, Droescher**
**Raleigh**
**North Carolina 27615 (US)**

(74) Representative: **Shenker, Paul Dhan**
**Novozymes A/S**
**Patents**
**Krogshoejvej 36**
**2880 Bagsvaerd (DK)**

(56) References cited:
**WO-A-2009/120931**

- **LAU ET AL: "Ethanolic fermentation of hydrolysates from ammonia fiber expansion (AFEX) treated corn stover and distillers grain without detoxification and external nutrient supplementation" BIOTECHNOLOGY AND BIOENGNEERING, vol. 99, 17 August 2007 (2007-08-17), pages 529-539, XP002522477**
- **"RESEARCH CHALLENGES AND OPPORTUNITIES FOR CELLULOSE CONVERSION TECHNOLOGY IN A DRY MILL PATHWAY" MIDWEST CONSORTIUM FOR BIOBASED PRODUCTS & BIOENERGY, [ONLINE], 2006, pages 1-40, XP002567971 Retrieved from the Internet: URL:http://cobweb.ecn.purdue.edu/~lorre/16/Midwest%20Consortium/Midwest%20Consort%20cellulose%20conv%20in%20dry%20mill%20as%20presented%20may%201%2006.pdf> [retrieved on 2010-03-11]**
- **DIEN ET AL: "Enzyme characterization for hydrolysis of AFEX and liquid hot-water pretreated distillers' grains and their conversion to ethanol" BIORESOURCE TECHNOLOGY, vol. 99, 8 November 2007 (2007-11-08), pages 5216-5225, XP022654841**
- **KIM ET AL: "Enzyme hydrolysis and ethanol fermentation of liquid hot water and AFEX pretreated distillers' grains at high-solids loadings" BIORESOURCE TECHNOLOGY, vol. 99, 19 November 2007 (2007-11-19), pages 5206-5215, XP022654840**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- HARRIS, P: "Genomics, Proteomics and Microarrays in Discovery MIC292 / Novozymes current efforts in enzymes for biomass utilization" [ONLINE], page 1,55, XP002567972 Retrieved from the Internet: URL: www.biotech.ucdavis.edu/PDFs/MIC292%20 Genomics%20&%20Proteomics%203-6-06.pdf> [retrieved on 2010-03-11]
- PERKIS ET AL: "Economic analysis of a modified dry grind ethanol process with recycle of pretreated and enzymatically hydrolyzed distillers' grains", BIORESOURCE TECHNOLOGY, vol. 99, 2008, pages 5243-5249, XP022654844,

## Description

### FIELD OF THE INVENTION

Methods for producing fermentation products from lignocellulose-containing material, and more particularly, a method for increasing the efficiency of producing fermentation products from lignocellulose-containing material by treating the material with distillers dried grains and/or distillers dried grains with solubles is disclosed. The methods include acid pretreatment and heating at a temperature in the range of 160-220 °C.

### BACKGROUND OF THE INVENTION

Lignocellulose-containing material, or biomass, may be used to produce fermentable sugars, which in turn may be used to produce fermentation products such as renewable fuels and chemicals. Lignocellulose-containing material is a complex structure of cellulose fibers wrapped in a lignin and hemicellulose sheath. Production of fermentation products from lignocellulose-containing material includes pre-treating, hydrolyzing, and fermenting the lignocellulose-containing material.

Conversion of lignocellulose-containing material into renewable fuels and chemicals often involves physical, biological, chemical and/or enzymatic treatment of the biomass with enzymes. In particular, enzymes hydrolyze cellulose to D-glucose, which is a simple fermentable sugar. In lignocellulose-containing material, high doses of enzyme are needed to degrade the cellulose to obtain high yields because lignin and lignin derivatives are believed to inhibit the enzyme(s) from hydrolyzing the cellulose. Such inhibition may occur in at least two ways: the lignin or lignin derivatives may preferentially bind with the enzyme thereby preventing the enzyme from binding with or hydrolyzing cellulose, and/or the lignin or lignin derivatives may cover portions of the cellulose thereby reducing enzyme access to cellulose. Consequently, when processing biomass, fewer enzymes may be available to degrade cellulose because the lignin or its derivatives may scavenge the enzyme or block its activity. Even for the enzymes that are available to degrade cellulose, the available enzyme may not be able to contact the cellulose because lignin may be covering the cellulose. Thus, the effectiveness of the process for digesting cellulose is reduced. In addition, the costs of enzymes are high. Thus, when the amount of enzymes needed to degrade cellulose is high, the processing costs are high and economically unfeasible.

Reduction in the amount of enzyme needed to obtain a satisfactory sugar yield can have a significant impact on process economics. Therefore, improving the efficiency of enzyme use is a major need in the bioconversion process. Several factors are thought to influence enzymatic hydrolysis of cellulose. These factors include lignin content, hemicellulose content, acetyl content, surface area of cellulose and cellulose crystallinity. It is generally understood that the lignin present in complex substrates has a negative effect on enzyme activity.

The exact role of lignin and lignin derivatives in limiting hydrolysis has been difficult to define. However, it is known that removing the effect of lignin and its derivatives increases hydrolysis of cellulose and increases fermentable sugar yield. This action may open more cellulose surface area for enzymatic attack and may reduce the amount of enzyme that is non-specifically adsorbed on the lignocellulosic substrate. Thus, compounds that remove the effect of lignin and its derivatives thereby making cellulose more accessible to enzymatic degradation are desirable.

Lau et al. Biotechnology and Bioengineering; Vol. 99, No. 3, February 15, 2008, pp. 529-539, concerns ethanolic fermentation of hydrolysates from Ammonia Fiber Expansion (AFEX) treated corn stover and Distillers Grain without detoxification and external nutrient supplementation.

Perkins et al (2008). Bioresource Technology 99, 2008, pp. 5243-5249, disclosed a modified dry grind ethanol process with recycle of pretreated and enzymatically hydrolyzed distillers' grains.

### SUMMARY OF THE INVENTION

Methods for producing fermentation products from lignocellulose-containing material by pre-treating and/or hydrolyzing the material in the presence of distillers dried grains and/or distillers dried grains with solubles are disclosed.

In one aspect the invention relates to methods for producing a fermentation product from a lignocellulose-containing material, comprising:

(a) combining lignocellulose-containing material and distillers dried grains with solubles (DDG/S);
(b) acid pre-treating the combined lignocellulose-containing material and DDG/S and heat treating at a temperature in the range of about 160-220°C for a period of 1-60 minutes;
(c) exposing the acid pre-treated and heat treated combined lignocellulose-containing material and DDG/S to one or more hydrolyzing enzymes; and
(d) fermenting with a fermenting organism to produce a fermentation product, wherein the DDG/S are introduced to the lignocellulose-containing material in an amount of 4-48% w/w DDG/S/lignocellulose-containing material.

In another aspect the invention relates to methods for enhancing enzymatic hydrolysis of a lignocellulose-containing material, comprising:

(a) introducing an effective lignin-blocking amount of DDG/S to the lignocellulose-containing material prior to acid pre- treatment and heat treatment at a temperature in the range of about 160-220°C for a period of 1-60 minutes, and
(b) exposing the acid pre-treated and heat treated lignocellulose-containing material and DDG/S to one or more hydrolyzing enzymes wherein the DDG/S are introduced to the lignocellulose-containing material in an amount of 4-48% w/w DDG/S/lignocellulose-containing material.

## BRIEF DESCRIPTION OF THE DRAWINGS

Figure 1. Effect of DDGs on enzymatic hydrolysis of dilute acid pre-treated corn stover with DDGs.
Figure 2. Effect of DDGs on cellulose conversion of dilute acid pre-treated corn stover with DDGs.

## DETAILED DESCRIPTION OF THE INVENTION

An improved and more efficient method for enzymatically hydrolyzing lignin containing biomass by using distillers dried grains with solubles (DDG/S) is disclosed. In one aspect the invention relates to methods for producing a fermentation product from a lignocellulose-containing material, comprising:

(a) combining lignocellulose-containing material and distillers dried grains with solubles (DDG/S);
(b) acid pre-treating the combined lignocellulose-containing material and DDG/S and heat treating at a temperature in the range of about 160-220°C for a period of 1-60 minutes;
(c) exposing the acid pre-treated and heat treated combined lignocellulose-containing material and DDG/S to one or more hydrolyzing enzymes; and
(d) fermenting with a fermenting organism to produce a fermentation product, wherein the DDG/S are introduced to the lignocellulose-containing material in an amount of 4-48% w/w DDG/S/lignocellulose-containing material.

Lignin is a phenolic polymer that can be derived by the dehydrogenative polymerization of coniferyl alcohol and/or sinapyl alcohol and is found in the cell walls of many plants. As used herein, the term "lignin" refers to the intact structure of the lignin polymer as well as any derivative fragments or compounds of the intact polymer that result from disruption of the lignin structure, including soluble lignin derivatives, condensed lignin and insoluble precipitated lignin. It is believed that lignin derivatives interact with DDG/S in a variety of ways. For example, insoluble lignin may directly combine with DDG/S and soluble lignin derivatives produced during pretreatment may be adsorbed by DDG/S.

As used herein, the term "biomass slurry" refers to the aqueous biomass material that undergoes enzymatic hydrolysis. Biomass slurry is produced by mixing biomass, e.g., corn stover, bagasse, etc., with water, buffer, and other pre-treatment materials. The biomass slurry may be pre-treated prior to hydrolysis.

As used herein, the term "lignin blocking" means the reduction or elimination of the deleterious effects of lignin on the process of converting biomass to a fermentation product. In addition, as used herein, the term "effective lignin blocking amount" means any amount useful in facilitating lignin blocking.

The method of the invention utilizes DDG/S, which it is believed may preferentially bind with lignin more readily than cellulose. Lignin containing biomass may be mixed with DDG/S, for example, by mixing DDG/S directly with dry biomass prior to the biomass being pre-treated. The combined biomass and DDG/S may then be slurried and pre-treated together. It is surmised that the DDG/S may preferentially bind with lignin in the combined slurry thereby covering lignin that has precipitated onto the surface of the cellulose, thus impeding the precipitated lignin from binding hydrolyzing enzyme. It is further surmised that DDG/S may be capable of adsorbing lignin that has not precipitated onto the cellulose surface. Cellulose-hydrolyzing enzymes may then hydrolyze cellulose more efficiently and rapidly. Without treatment of the lignin-containing biomass with DDG/S, lignin may bind a portion of the cellulose-hydrolyzing enzymes rendering them unable to hydrolyze cellulose, or may cover portions of the cellulose, rendering it inaccessible to hydrolyzing enzymes.

Without being bound by any particular theory, it is believed that lignin operates in multiple ways to inhibit enzymes from hydrolyzing cellulose in biomass. Lignin limits the degree to which cellulose can be converted to monomeric sugars by cellulolytic and hemicellulolytic enzymes. The focus of many research activities has been directed to understanding the nature of lignin in cell walls and developing pre-treatment processes that are effective in removing it. By understanding the mode in which lignin inhibits enzymatic activity, it is possible to reduce the detrimental effects traditionally caused by lignin in biomass. Lignocellulose-containing material or biomass may be acid pre-treated prior to being

hydrolyzed. Acid pre-treatment chemically alters the lignin component of the biomass, forming lignin derivatives including condensed lignin that precipitates on the cellulose fiber surface. The condensed lignin may inhibit enzymes from reaching the cellulose by covering the cellulose fiber surface. Other lignin derivatives formed during acid pre-treatment include small phenol containing fragments and compounds that may inhibit enzyme function.

It is further believed that treatment of biomass with DDG/S is effective, at least in part, through binding lignin, thus reducing and/or inhibiting non-productive adsorption of hydrolyzing enzymes to lignin. The treatment of biomass with DDG/S may improve processing of lignin containing substrates by inhibiting lignin from binding to the enzymes and improving activity of the enzyme. DDG/S may reduce enzyme load and/or improve enzyme performance because the enzymes may not become bound to the lignin thus remaining available to more effectively hydrolyze the biomass substrate.

It is believed that the present method reduces enzyme loading in hydrolysis of biomass. The amount of enzyme that is needed to provide hydrolysis is significantly reduced through treating the biomass with DDG/S. Reduction in enzyme loading reduces the overall costs of the biomass conversion processes.

The invention also relates to methods for enhancing enzymatic hydrolysis of a lignocellulose-containing material, comprising:

(a) introducing an effective lignin-blocking amount of DDG/S to the lignocellulose-containing material prior to acid pre- treatment and heat treatment at a temperature in the range of about 160-220°C for a period of 1-60 minutes, and
(b) exposing the acid pre-treated and heat treated lignocellulose-containing material and DDG/S to one or more hydrolyzing enzymes wherein the DDG/S are introduced to the lignocellulose-containing material in an amount of 4-48% w/w DDG/S/lignocellulose-containing material.

As indicated, it is contemplated that the DDG/S may be added to dry biomass. The combined biomass and DDG/S may then be slurried and pre-treated together. The combined, pre-treated biomass slurry may then be washed to aid in removal of soluble lignin. The washed biomass slurry may then be exposed to hydrolyzing enzyme. Typically, any lignin blocking additive is added after pre-treatment. The lignin blocking additive is pre-treated separately from the biomass slurry thereby resulting in two pre-treatment processes, one for the biomass and one for the additive. Thus, it is advantageous to add DDG/S to dry, biomass prior to slurry and pre-treatment because the biomass and DDG/S may be pre-treated together thus removing the need for a pre-treatment step for the biomass and DDG/S separately.

<u>Distillers Dried Grains with or without Solubles (DDG/S)</u>

Distiller's dried grains with or without solubles (DDG/S) is a co-product of the distillery industries. Most DDG/S in North America comes from plants that produce ethanol for oxygenated fuels, and the remaining portion of DDG/S is produced by the alcohol beverage industry.

Distiller's dried grains with or without solubles are the dried residue remaining after the starch fraction of corn is fermented with selected yeasts and enzymes to produce alcohol and carbon dioxide. After complete fermentation, the alcohol is removed by distillation and the remaining fermentation residues are dried.

A third of the grain that goes into ethanol production comes out as DDG/S. Generally each bushel of grain used in the ethanol-making process produces about 2.7 gallons (10.2 liters) of ethanol; about 18 pounds (8.2 kg) of DDG/S, and about 18 pounds (8.2 kg) of carbon dioxide.

DDG/S is used as animal feed. It is rich in cereal and residual yeast proteins, energy, minerals and vitamins. It is an excellent digestible protein and energy source for beef cattle. It can also be used in turkey and swine applications. DDG/S generally comprises about 30% crude protein, about 10% fat, about 6% fiber, and about 50% carbohydrates.

DDG/S are further economically advantageous to the bioconversion process because in addition to the cellulose from the lignocellulose-containing material being more efficiently hydrolyzed into fermentable sugars, a portion of the DDG/S can be converted to glucose. Thus, in addition to indirectly contributing to sugar yield by inhibiting the detrimental effects of lignin, DDG/S directly contribute to sugar yield through conversion of the carbohydrate component. It is contemplated that amylase may be added to the biomass slurry after some period of hydrolysis, e.g., after 72 hours of hydrolysis, in order to convert the carbohydrates in DDG/S to glucose, especially wherein acid pre-treatment methods have been employed. An additional economic advantage to using DDG/S is that the pre-treatment of DDG/S results in the release of polypeptides. Certain fermenting organisms such as yeast require adequate nitrogen for propagation and fermentation. The polypeptides released from the DDG/S can provide the required nitrogen such that another exogenous source is not necessary.

As indicated previously, an additional benefit to using DDG/S is that it may be added to dry biomass prior to slurrying and pre-treatment. Foregoing an extra pre-treatment step for the DDG/S additive is economically advantageous in the biomass conversion process.

It is envisioned that first mixing dry biomass with DDG/S, then slurrying and pre-treating the mixture, and then

adding cellulose hydrolyzing enzyme to the mixture, provides the highest efficiency in cellulose conversion. Because DDG/S comprise carbohydrates that may themselves be hydrolyzed, it is possible to hydrolyze a portion of the DDG/S itself, thereby further increasing sugar production. Treating biomass with DDG/S produces a hydrolysis yield from cellulose that may be measured as percentage improvement in final sugar yield or cellulose conversion rate. By way of example, an approximately 12% improvement in final sugar yield may be obtained in comparison to the hydrolysis yield from cellulose of a biomass slurry that is not treated with DDG/S. In addition, by way of further example, an approximately 12% improvement in cellulose conversion rate may be obtained in comparison to hydrolysis yield from cellulose of a biomass slurry that is not treated with DDG/S.

Without being bound by any particular theory, it is believed that nonspecific binding of DDG/S to lignin may decrease unproductive binding of enzymes to lignin surfaces or inhibition of enzyme activity due to interactions with lignin. Thus, use of DDG/S in a process for lignocellulose conversion advantageously facilitates a lowering of the enzyme loading level to achieve the same target conversion percentage.

Lignocellulose-Containing Material

"Lignocellulose" or "lignocellulose-containing material" means material primarily consisting of cellulose, hemicellulose, and lignin. Such material is often referred to as "biomass."

Biomass is a complex structure of cellulose fibers wrapped in a lignin and hemicellulose sheath. The structure of biomass is such that it is not susceptible to enzymatic hydrolysis. In order to enhance enzymatic hydrolysis, the biomass has to be pre-treated, e.g., by acid hydrolysis under adequate conditions of pressure and temperature, in order to break the lignin seal, saccharify and solubilize the hemicellulose, and disrupt the crystalline structure of the cellulose. The cellulose can then be hydrolyzed enzymatically, e.g., by cellulolytic enzyme treatment, to convert the carbohydrate polymers into fermentable sugars which may be fermented into a desired fermentation product, such as ethanol. Hemicellulolytic enzyme treatments may also be employed to hydrolyze any remaining hemicellulose in the pre-treated biomass.

The biomass may be any material containing lignocellulose. In a preferred embodiment, the biomass contains at least about 30 wt. %, preferably at least about 50 wt. %, more preferably at least about 70 wt. %, even more preferably at least about 90 wt. %, lignocellulose. It is to be understood that the biomass may also comprise other constituents such as proteinaceous material, starch, and sugars such as fermentable or un-fermentable sugars or mixtures thereof.

Biomass is generally found, for example, in the stems, leaves, hulls, husks, and cobs of plants or leaves, branches, and wood of trees. Biomass includes, but is not limited to, herbaceous material, agricultural residues, forestry residues, municipal solid wastes, waste paper, and pulp and paper mill residues. It is to be understood that biomass may be in the form of plant cell wall material containing lignin, cellulose, and hemicellulose in a mixed matrix.

Other examples of suitable biomass include corn fiber, rice straw, pine wood, wood chips, bagasse, paper and pulp processing waste, corn stover, corn cobs, hard wood such as poplar and birch, soft wood, cereal straw such as wheat straw, rice straw, switch grass, Miscanthus, rice hulls, municipal solid waste (MSW), industrial organic waste, office paper, or mixtures thereof.

In a preferred embodiment, the biomass is selected from one or more of corn stover, corn cobs, corn fiber, wheat straw, rice straw, switch grass, and bagasse.

Pre-treatment

In accordance with the present invention, acid pre-treatment may include the introduction of DDG/S or a similar compound to the dry biomass prior to slurrying or what is considered to be conventional pre-treatment.

Pre-treatment is carried out before hydrolysis or fermentation. The goal of pre-treatment is to separate or release cellulose, hemicellulose, and lignin and thus improving the rate or efficiency of hydrolysis. The acid pre-treatment step may include a step wherein DDG/S are added to the biomass. As indicated previously, the biomass is typically in a dry form when DDG/S are added. The combined biomass and DDG/S are then subjected to conventional slurrying and acid pre-treatment It further comprises heat treatment. DDG/S are added in an amount of about 4-48 wt. % dry biomass. The biomass may be present during pre-treatment in an amount between about 10-80 wt. %, preferably between about 20-70 wt. %, especially between about 30-60 wt. %, such as around about 50 wt. %.According to the invention the pre-treatment is acid treatment, more preferably, a continuous dilute or mild acid treatment such as treatment with sulfuric acid, or another organic acid such as acetic acid, citric acid, tartaric acid, succinic acid, hydrogen chloride or mixtures thereof. Other acids may also be used. Mild acid treatment means that the treatment pH lies in the range from about pH 1-5, preferably about pH 1-3. In a specific embodiment the acid concentration is in the range from 0.1 to 2.0 wt. % acid and is preferably sulfuric acid. The acid may be contacted with the biomass and the mixture may be held at a temperature in the range of about 160-220°C, such as about 165-195°C, for periods ranging from minutes to seconds, e.g., 1-60 minutes, such as 2-30 minutes or 3-12 minutes. Addition of strong acids such as sulfuric acid may be applied to remove

hemicellulose. The addition of strong acids enhances the digestibility of cellulose.

The acid pre-treatment step may involve dilute or mild acid treatment and high temperature and/or pressure treatment. The pre-treatments may be carried out sequentially or simultaneously, as desired.

In a preferred embodiment pre-treatment is carried out as a dilute or mild acid pre-treatment step.

Hydrolysis

Before the acid pre-treated biomass, preferably in the form of biomass slurry, is fermented it may be hydrolyzed to break down cellulose and hemicellulose into fermentable sugars. In a preferred embodiment, the pre-treated material is hydrolyzed, preferably enzymatically, before fermentation.

The dry solids content during hydrolysis may be in the range from about 5-50 wt. %, preferably about 10-40 wt. %, preferably about 20-30 wt. %. Hydrolysis may in a preferred embodiment be carried out as a fed batch process where the pre-treated biomass (*i.e.*, the substrate) is fed gradually to, *e.g.*, an enzyme containing hydrolysis solution.

In a preferred embodiment hydrolysis is carried out enzymatically. According to the invention, the pre-treated biomass slurry may be hydrolyzed by one or more cellulolytic enzymes, such as cellulases or hemicellulases, or combinations thereof.

In a preferred embodiment, hydrolysis is carried out using a cellulolytic enzyme preparation comprising one or more polypeptides having cellulolytic enhancing activity. In a preferred embodiment, the polypeptide(s) having cellulolytic enhancing activity is of family GH61A origin. Examples of suitable and preferred cellulolytic enzyme preparations and polypeptides having cellulolytic enhancing activity are described in the "Cellulolytic Enzymes" section below.

As the biomass may contain constituents other than lignin, cellulose and hemicellulose, hydrolysis and/or fermentation may be carried out in the presence of additional enzyme activities such as protease activity, amylase activity, carbohydrate-generating enzyme activity, and esterase activity such as lipase activity.

Enzymatic hydrolysis is preferably carried out in a suitable aqueous environment under conditions which can readily be determined by one skilled in the art. In a preferred embodiment, hydrolysis is carried out at suitable, preferably optimal, conditions for the enzyme(s) in question.

Suitable process time, temperature and pH conditions can readily be determined by one skilled in the art. Preferably, hydrolysis is carried out at a temperature between 25 and 70°C, preferably between 40 and 60°C, especially around 50°C. Hydrolysis is preferably carried out at a pH in the range from pH 3-8, preferably pH 4-6, especially around pH 5. In addition, hydrolysis is typically carried out for between 12 and 96 hours, preferably 16 to 72 hours, more preferably between 24 and 48 hours.

Fermentation

Fermentable sugars from pre-treated and/or hydrolyzed biomass may be fermented by one or more fermenting organisms capable of fermenting sugars, such as glucose, xylose, mannose, and galactose directly or indirectly into a desired fermentation product. The fermentation conditions depend on the desired fermentation product and fermenting organism and can easily be determined by one of ordinary skill in the art.

Especially in the case of ethanol fermentation, the fermentation may be ongoing for between 1-48 hours, preferably 1-24 hours. In an embodiment, the fermentation is carried out at a temperature between about 20 to 40°C, preferably about 26 to 34°C, in particular around 32°C. In one embodiment, the pH is greater than 5. In another embodiment, the pH is from about pH 3-7, preferably 4-6. However, some, *e.g.*, bacterial fermenting organisms have higher fermentation temperature optima. Therefore, in an embodiment, the fermentation is carried out at temperature between about 40-60°C, such as 50-60°C. The skilled person in the art can easily determine suitable fermentation conditions.

Fermentation can be carried out in a batch, fed-batch, or continuous reactor. Fed-batch fermentation may be fixed volume or variable volume fed-batch. In one embodiment, fed-batch fermentation is employed. The volume and rate of fed-batch fermentation depends on, for example, the fermenting organism, the identity and concentration of fermentable sugars, and the desired fermentation product. Such fermentation rates and volumes can readily be determined by one of ordinary skill in the art.

SSF, HHF and SHF

Hydrolysis and fermentation may be carried out as a simultaneous hydrolysis and fermentation step (SSF). In general, this means that combined/simultaneous hydrolysis and fermentation are carried out at conditions (*e.g.*, temperature and/or pH) suitable, preferably optimal, for the fermenting organism(s) in question.

The hydrolysis step and fermentation step may be carried out as hybrid hydrolysis and fermentation (HHF). HHF typically begins with a separate partial hydrolysis step and ends with a simultaneous hydrolysis and fermentation step. The separate partial hydrolysis step is an enzymatic cellulose saccharification step typically carried out at conditions

(*e.g.*, at higher temperatures) suitable, preferably optimal, for the hydrolyzing enzyme(s) in question. The subsequent simultaneous hydrolysis and fermentation step is typically carried out at conditions suitable for the fermenting organism(s) (often at lower temperatures than the separate hydrolysis step).

The hydrolysis and fermentation steps may also be carried out as separate hydrolysis and fermentation, where the hydrolysis is taken to completion before initiation of fermentation. This is often referred to as "SHF".

Recovery

Subsequent to fermentation, the fermentation product may optionally be separated from the fermentation medium in any suitable way. For instance, the medium may be distilled to extract the fermentation product, or the fermentation product may be extracted from the fermentation medium by micro or membrane filtration techniques. Alternatively, the fermentation product may be recovered by stripping. Recovery methods are well known in the art.

Fermentation Products

The present invention may be used for producing any fermentation product. Preferred fermentation products include alcohols (*e.g.*, ethanol, methanol, butanol); organic acids (*e.g.*, citric acid, acetic acid, itaconic acid, lactic acid, gluconic acid); ketones (*e.g.*, acetone); amino acids (*e.g.*, glutamic acid); gases (*e.g.*, $H_2$ and $CO_2$); antibiotics (*e.g.*, penicillin and tetracycline); enzymes; vitamins (*e.g.*, riboflavin, B12, beta-carotene); and hormones.

Other products include consumable alcohol industry products, *e.g.*, beer and wine; dairy industry products, *e.g.*, fermented dairy products; leather industry products and tobacco industry products. In a preferred embodiment, the fermentation product is an alcohol, especially ethanol. The fermentation product, such as ethanol, obtained according to the invention, may preferably be used as fuel alcohol/ethanol. However, in the case of ethanol, it may also be used as potable ethanol.

Fermenting Organism

The phrase "fermenting organism" refers to any organism, including bacterial and fungal organisms, suitable for producing a desired fermentation product. The fermenting organism may be C6 or C5 fermenting organisms, or a combination thereof. Both C6 and C5 fermenting organisms are well known in the art.

Suitable fermenting organisms are able to ferment, *i.e.*, convert, fermentable sugars, such as glucose, fructose, maltose, xylose, mannose and or arabinose, directly or indirectly into the desired fermentation product.

Examples of fermenting organisms include fungal organisms such as yeast. Preferred yeast includes strains of the genus *Saccharomyces,* in particular strains of *Saccharomyces cerevisiae* or *Saccharomyces uvarum;* a strain of *Pichia,* preferably *Pichia stipitis* such as *Pichia stipitis* CBS 5773 or *Pichia pastoris;* a strain of the genus *Candida,* in particular a strain of *Candida utilis, Candida arabinofermentans, Candida diddensii, Candida sonorensis, Candida shehatae, Candida tropicalis,* or *Candida boidinii.* Other fermenting organisms include strains of *Hansenula,* in particular *Hansenula polymorpha* or *Hansenula anomala; Kluyveromyces,* in particular *Kluyveromyces fragilis* or *Kluyveromyces marxianus;* and *Schizosaccharomyces,* in particular *Schizosaccharomyces pombe.*

Preferred bacterial fermenting organisms include strains of *Escherichia,* in particular *Escherichia coli,* strains of *Zymomonas,* in particular *Zymomonas mobilis,* strains of *Zymobacter,* in particular *Zymobactor palmae,* strains of *Klebsiella* in particular *Klebsiella* oxytoca, strains of *Leuconostoc,* in particular *Leuconostoc mesenferoides,* strains of *Clostridium,* in particular *Clostridium butyricum,* strains of *Enterobacter,* in particular *Enterobacter aerogenes* and strains of *Thermoanaerobacter,* in particular *Thermoanaerobacter* BG1L1 *(*Appl. Microbiol. Biotech. 77: 61-86) and *Thermoanarobacter ethanolicus, Thermoanaerobacter thermosaccharolyticum,* or *Thermoanaerobacter mathranii.* Strains of *Lactobacillus* are also envisioned as are strains of *Corynebacterium glutamicum R, Bacillus thermoglucosidaisus,* and *Geobacillus thermoglucosidasius.*

In an embodiment the fermenting organism is a C6 sugar fermenting organism, such as a strain of, *e.g., Saccharomyces cerevisiae.*

In connection with fermentation of lignocellulose derived materials, C5 sugar fermenting organisms are contemplated. Most C5 sugar fermenting organisms also ferment C6 sugars. Examples of C5 sugar fermenting organisms include strains of *Pichia,* such as of the species *Pichia stipitis.* C5 sugar fermenting bacteria are also known. Also some *Saccharomyces cerevisae* strains ferment C5 (and C6) sugars. Examples are genetically modified strains of *Saccharomyces spp.* that are capable of fermenting C5 sugars include the ones concerned in, *e.g.*, Ho et al., 1998, Applied and Environmental Microbiology, p. 1852-1859 and Karhumaa et al., 2006, Microbial Cell Factories 5:18, and Kuyper et al., 2005, FEMS Yeast Research 5, p. 925-934.

Certain fermenting organisms' fermentative performance may be inhibited by the presence of inhibitors in the fermentation media and thus reduce ethanol production capacity. Compounds in biomass hydrosylates and high concentrations of ethanol are known to inhibit the fermentative capacity of certain yeast cells. Pre-adaptation or adaptation methods may reduce this inhibitory effect. Typically pre-adaptation or adaptation of yeast cells involves sequentially growing yeast cells, prior to fermentation, to increase the fermentative performance of the yeast and increase ethanol production. Methods of yeast pre-adaptation and adaptation are known in the art. Such methods may include, for example, growing the yeast cells in the presence of crude biomass hydrolyzates; growing yeast cells in the presence of inhibitors such as phenolic compounds, furaldehydes and organic acids; growing yeast cells in the presence of non-inhibiting amounts of ethanol; and supplementing the yeast cultures with acetaldehyde. In one embodiment, the fermenting organism is a yeast strain subject to one or more pre-adaptation or adaptation methods prior to fermentation.

Certain fermenting organisms such as yeast require an adequate source of nitrogen for propagation and fermentation. Many sources of nitrogen can be used and such sources of nitrogen are well known in the art. In one embodiment, a low cost source of nitrogen is used. Such low cost sources can be organic, such as urea, DDGs, wet cake or corn mash, or inorganic, such as ammonia or ammonium hydroxide.

Commercially available yeast suitable for ethanol production includes, *e.g.*, ETHANOL RED™ yeast (available from Fermentis/Lesaffre, USA), FALI™ (available from Fleischmann's Yeast, USA), SUPERSTART and THERMOSACC™ fresh yeast (available from Ethanol Technology, WI, USA), BIOFERM AFT and XR (available from NABC - North American Bioproducts Corporation, GA, USA), GERT STRAND (available from Gert Strand AB, Sweden), and FERMIOL (available from DSM Specialties).

<u>Enzymes</u>

Even if not specifically mentioned in the context of a method or process of the invention, it is to be understood that the enzyme(s) as well as other compounds are used in an effective amount. One or more enzymes may be used.

The phrase "cellulolytic activity" as used herein is understood as comprising enzymes having cellobiohydrolase activity (EC 3.2.1.91), e.g., cellobiohydrolase I and cellobiohydrolase II, as well as endo-glucanase activity (EC 3.2.1.4) and beta-glucosidase activity (EC 3.2.1.21).

The cellulolytic activity may, in a preferred embodiment, be in the form of a preparation of enzymes of fungal origin, such as from a strain of the genus *Trichoderma,* preferably a strain of *Trichoderma reesei;* a strain of the genus *Humicola,* such as a strain of *Humicola insolens;* or a strain of *Chrysosporium,* preferably a strain of *Chrysosporium lucknowense.*

The cellulolytic enzyme preparation may contain one or more of the following activities: enzyme, hemienzyme, cellulolytic enzyme enhancing activity, beta-glucosidase activity, endoglucanase, cellubiohydrolase, or xylose isomerase.

The enzyme may be a composition as defined in PCT/US2008/065417. For example, the cellulolytic enzyme preparation comprises a polypeptide having cellulolytic enhancing activity, preferably a family GH61A polypeptide, preferably the one disclosed in WO 2005/074656 (Novozymes). The cellulolytic enzyme preparation may further comprise a beta-glucosidase, such as a beta-glucosidase derived from a strain of the genus *Trichoderma, Aspergillus* or *Penicillium,* including the fusion protein having beta-glucosidase activity disclosed in WO 2008/057637. The cellulolytic enzyme preparation may also comprise a CBH 11 enzyme, preferably *Thielavia terrestris* cellobiohydrolase II CEL6A. The cellulolytic enzyme preparation may also comprise cellulolytic enzymes, preferably one derived from *Trichoderma reesei* or *Humicola insolens.*

The cellulolytic enzyme preparation may also comprising a polypeptide having cellulolytic enhancing activity (GH61A) disclosed in WO 2005/074656; a beta-glucosidase (fusion protein disclosed in WO 2008/057637) and cellulolytic enzymes derived from *Trichoderma reesei.*

The cellulolytic enzyme may be the commercially available product CELLUCLAST® 1.5L or CELLUZYME™ available from Novozymes A/S, Denmark or ACCELERASE™ 1000 (from Genencor Inc., USA).

A cellulolytic enzyme may be added for hydrolyzing pre-treated biomass slurry. The cellulolytic enzyme may be dosed in the range from 0.1-100 FPU per gram total solids (TS), preferably 0.5-50 FPU per gram TS, especially 1-20 FPU per gram TS. In another embodiment, at least 0.1 mg cellulolytic enzyme per gram total solids (TS), preferably at least 3 mg cellulolytic enzyme per gram TS, such as between 5 and 10 mg cellulolytic enzyme(s) per gram TS is(are) used for hydrolysis.

<u>Endoglucanase (EG)</u>

One or more endoglucanases may be present during hydrolysis. The term "endoglucanase" means an endo-1,4-(1,3;1,4)-beta-D-glucan 4-glucanohydrolase (E.C. No. 3.2.1.4), which catalyses endo-hydrolysis of 1,4-beta-D-glycosidic linkages in cellulose, cellulose derivatives (such as carboxymethyl cellulose and hydroxyethyl cellulose), lichenin, beta-1,4 bonds in mixed beta-1,3 glucans such as cereal beta-D-glucans or xyloglucans, and other plant material containing cellulosic components. Endoglucanase activity may be determined using carboxymethyl cellulose (CMC) hydrolysis according to the procedure of Ghose, 1987, Pure and Appl. Chem. 59: 257-268.

Endoglucanases may be derived from a strain of the genus *Trichoderma,* preferably a strain of *Trichoderma reesei;* a strain of the genus *Humicola,* such as a strain of *Humicola insolens;* or a strain of *Chrysosporium,* preferably a strain of *Chrysosporium lucknowense.*

Cellobiohydrolase (CBH)

One or more cellobiohydrollases may be present during hydrolysis. The term "cellobiohydrolase" means a 1,4-beta-D-glucan cellobiohydrolase (E.C. 3.2.1.91), which catalyzes the hydrolysis of 1,4-beta-D-glucosidic linkages in cellulose, cellooligosaccharides, or any beta-1,4-linked glucose containing polymer, releasing cellobiose from the reducing or non-reducing ends of the chain.

Examples of cellobiohydroloses are mentioned above including CBH I and CBH II from *Trichoderma reseei; Humicola insolens* and CBH II from *Thielavia terrestris* cellobiohydrolase (CELL6A).

Cellobiohydrolase activity may be determined according to the procedures described by Lever et al., 1972, Anal. Biochem. 47: 273-279 and by van Tilbeurgh et al., 1982, FEBS Letters 149: 152-156; van Tilbeurgh and Claeyssens, 1985, FEBS Letters 187: 283-288. The Lever *et al.* method is suitable for assessing hydrolysis of cellulose in corn stover and the method of van Tilbeurgh *et al.* is suitable for determining the cellobiohydrolase activity on a fluorescent disaccharide derivative.

Beta-glucosidase

One or more beta-glucosidases may be present during hydrolysis. The term "beta-glucosidase" means a beta-D-glucoside glucohydrolase (E.C. 3.2.1.21), which catalyzes the hydrolysis of terminal non-reducing beta-D-glucose residues with the release of beta-D-glucose. For purposes of the present invention, beta-glucosidase activity is determined according to the basic procedure described by Venturi et al., 2002, J. Basic Microbiol. 42: 55-66, except different conditions were employed as described herein. One unit of beta-glucosidase activity is defined as 1.0 μmole of p-nitrophenol produced per minute at 50°C, pH 5 from 4 mM p-nitrophenyl-beta-D-glucopyranoside as substrate in 100 mM sodium citrate, 0.01% TWEEN® 20.

The beta-glucosidase may be of fungal origin, such as a strain of the genus *Trichoderma, Aspergillus* or *Penicillium.* The beta-glucosidase may be derived from *Trichoderma reesei,* such as the beta-glucosidase encoded by the *bgl1* gene (see Fig. 1 of EP 562003). The beta-glucosidase may be derived from *Aspergillus oryzae* (recombinantly produced in *Aspergillus oryzae* according to WO 2002/095014), *Aspergillus fumigatus* (recombinantly produced in *Aspergillus oryzae* according to Example 22 of WO 2002/095014) or *Aspergillus niger* (1981, J. Appl. Vol 3, pp 157-163).

Hemicellulase

Hemicellulose can be broken down by hemienzymes and/or acid hydrolysis to release its five and six carbon sugar components. The lignocellulose derived material may be treated with one or more hemicellulases. Any hemicellulase suitable for use in hydrolyzing hemicellulose, preferably into xylose, may be used.

Preferred hemicellulases include xylanases, arabinofuranosidases, acetyl xylan esterase, feruloyl esterase, glucuronidases, endo-galactanase, mannases, endo or exo arabinases, exo-galactanses, and mixtures of two or more thereof. Preferably, the hemicellulase for use in the present invention is an exo-acting hemicellulase, and more preferably, the hemicellulase is an exo-acting hemicellulase which has the ability to hydrolyze hemicellulose under acidic conditions of below pH 7, preferably pH 3-7. An example of hemicellulase suitable for use in the present invention includes VISCOZYME™ (available from Novozymes A/S, Denmark).

The hemicellulase may be a xylanase. The xylanase may preferably be of microbial origin, such as of fungal origin (*e.g., Trichoderma, Meripilus, Humicola, Aspergillus, Fusarium*) or from a bacterium (*e.g., Bacillus*). The xylanase may be derived from a filamentous fungus, preferably derived from a strain of *Aspergillus,* such as *Aspergillus aculeatus;* or a strain of *Humicola,* preferably *Humicola lanuginosa.* The xylanase may preferably be an endo-1,4-beta-xylanase, more preferably an endo-1,4-beta-xylanase of GH10 or GH11. Examples of commercial xylanases include SHEARZYME™ and BIOFEED WHEAT™ from Novozymes A/S, Denmark.

The hemicellulase may be added in an amount effective to hydrolyze hemicellulose, such as, in amounts from about 0.001 to 0.5 wt. % of total solids (TS), more preferably from about 0.05 to 0.5 wt. % of TS.

Xylanases may be added in amounts of 0.001-1.0 g/kg DM (dry matter) substrate, preferably in the amounts of 0.005-0.5 g/kg DM substrate, and most preferably from 0.05-0.10 g/kg DM substrate.

Xylose Isomerase

Xylose isomerases (D-xylose ketoisomerase) (E.C. 5.3.1.5.) are enzymes that catalyze the reversible isomerization reaction of D-xylose to D-xylulose. Glucose isomerases convert the reversible isomerization of D-glucose to D-fructose. However, glucose isomarase is sometimes referred to as xylose isomerase.

A xylose isomerase may be used in the method or process and may be any enzyme having xylose isomerase activity and may be derived from any sources, preferably bacterial or fungal origin, such as filamentous fungi or yeast. Examples of bacterial xylose isomerases include the ones belonging to the genera *Streptomyces, Actinoplanes, Bacillus* and *Flavobacterium,* and *Thermotoga,* including *T. neapolitana* (Vieille et al., 1995, Appl. Environ. Microbiol. 61 (5), 1867-1875) and *T. maritime.* Examples of fungal xylose isomerases are derived species of *Basidiomycetes.*

A preferred xylose isomerase is derived from a strain of yeast genus *Candida,* preferably a strain of *Candida boidinii,* especially the *Candida boidinii* xylose isomerase disclosed by, *e.g.,* Vongsuvanlert et al., 1988, Agric. Biol. Chem., 52(7): 1817-1824. The xylose isomerase may preferably be derived from a strain of *Candida boidinii* (*Kloeckera 2201*), deposited as DSM 70034 and ATCC 48180, disclosed in Ogata et al., Agric. Biol. Chem, Vol. 33, p. 1519-1520 or Vongsuvanlert et al., 1988, Agric. Biol. Chem, 52(2), p. 1519-1520.

In one embodiment, the xylose isomerase is derived from a strain of *Streptomyces, e.g*., derived from a strain of *Streptomyces murinus* (U.S. Patent No. 4,687,742); *S. flavovirens, S. albus, S. achromogenus, S. echinatus, S. wedmorensis* all disclosed in U.S. Patent No. 3,616,221. Other xylose isomerases are disclosed in U.S. Patent No. 3,622,463, U.S. Patent No. 4,351,903, U.S. Patent No. 4,137,126, U.S. Patent No. 3,625,828, HU patent no. 12,415, DE patent 2,417,642, JP patent no. 69,28,473, and WO 2004/044129. The xylose isomerase may be either in immobilized or liquid form. Liquid form is preferred. Examples of commercially available xylose isomerases include SWEETZYME™ T from Novozymes A/S, Denmark. The xylose isomerase is added in an amount to provide an activity level in the range from 0.01-100 IGIU per gram total solids.

Alpha-Amylase

One or more alpha-amylases may be used. Preferred alpha-amylases are of microbial, such as bacterial or fungal origin. The most suitable alpha-amylase is determined based on process conditions but can easily be done by one skilled in the art.

The preferred alpha-amylase may be an acid alpha-amylase, *e.g*., fungal acid alpha-amylase or bacterial acid alpha-amylase. The phrase "acid alpha-amylase" means an alpha-amylase (E.C. 3.2.1.1) which added in an effective amount has activity optimum at a pH in the range of 3 to 7, preferably from 3.5 to 6, or more preferably from 4-5.

Bacterial Alpha-Amylase

As indicated above, the alpha-amylase may be of *Bacillus* origin. The *Bacillus* alpha-amylase may preferably be derived from a strain of *B. licheniformis, B. amyloliquefaciens, B. subtilis* or *B. stearothermophilus,* but may also be derived from other *Bacillus* sp. Specific examples of contemplated alpha-amylases include the *Bacillus licheniformis* alpha-amylase shown in SEQ ID NO: 4 in WO 1999/19467, the *Bacillus amyloliquefaciens* alpha-amylase SEQ ID NO: 5 in WO 1999/19467 and the *Bacillus stearothermophilus* alpha-amylase shown in SEQ ID NO: 3 in WO 1999/19467. In an embodiment, the alpha-amylase may be an enzyme having a degree of identity of at least 60%, preferably at least 70%, more preferred at least 80%, even more preferred at least 90%, such as at least 95%, at least 96%, at least 97%, at least 98% or at least 99% to any of the sequences shown in SEQ ID NO: 1, 2 or 3, respectively, in WO 1999/19467.

The *Bacillus* alpha-amylase may also be a variant and/or hybrid, especially one described in any of WO 1996/23873, WO 1996/23874, WO 1997/41213, WO 1999/19467, WO 2000/60059, and WO 2002/10355. Specifically contemplated alpha-amylase variants are disclosed in U.S. Patent No. 6,093,562, 6,297,038 or 6,187,576 and include *Bacillus stearothermophilus* alpha-amylase (BSG alpha-amylase) variants having a deletion of one or two amino acid in positions R179 to G182, preferably a double deletion disclosed in WO 1996/023873 - see *e.g*., page 20, lines 1-10, preferably corresponding to delta(181-182) compared to the wild-type BSG alpha-amylase amino acid sequence set forth in SEQ ID NO: 3 disclosed in WO 1999/19467 or deletion of amino acids R179 and G180 using SEQ ID NO: 3 in WO 1999/19467 for numbering. Even more preferred are *Bacillus* alpha-amylases, especially *Bacillus stearothermophilus* alpha-amylase, which have a double deletion corresponding to delta(181-182) and further comprise a N193F substitution (also denoted 1181* + G182* + N193F) compared to the wild-type BSG alpha-amylase amino acid sequence set forth in SEQ ID NO:3 disclosed in WO 1999/19467.

Bacterial Hybrid Alpha-Amylase

One or more bacterial hybrid alpha-amylases may be used. A hybrid alpha-amylase specifically contemplated comprises 445 C-terminal amino acid residues of the *Bacillus licheniformis* alpha-amylase (shown in SEQ ID NO: 4 of WO 1999/19467) and the 37 N-terminal amino acid residues of the alpha-amylase derived from *Bacillus amyloliquefaciens* (shown in SEQ ID NO: 5 of WO 1999/19467), with one or more, especially all, of the following substitution:

48A+T49I+G107A+H156Y+A181T+N190F+I201F+A209V+Q264S (using the *Bacillus licheniformis* numbering in SEQ ID NO: 4 of WO 1999/19467). Also preferred are variants having one or more of the following mutations (or corresponding mutations in other *Bacillus* alpha-amylase backbones): H154Y, A181T, N190F, A209V and Q264S and/or deletion of two residues between positions 176 and 179, preferably deletion of E178 and G179 (using the SEQ ID NO: 5 numbering of WO 1999/19467).

Fungal Alpha-Amylase

One or more fungal alpha-amylases may be used. Fungal alpha-amylases include alpha-amylases derived from a strain of the genus *Aspergillus,* such as, *Aspergillus oryzae, Aspergillus niger* and *Aspergillis kawachii* alpha-amylases.

A preferred acidic fungal alpha-amylase is a Fungamyl-like alpha-amylase, which is derived from a strain of *Aspergillus oryzae.* The phrase "Fungamyl-like alpha-amylase" indicates an alpha-amylase which exhibits a high identity, i.e., more than 70%, more than 75%, more than 80%, more than 85% more than 90%, more than 95%, more than 96%, more than 97%, more than 98%, more than 99% or even 100% identity to the mature part of the amino acid sequence shown in SEQ ID NO: 10 in WO 1996/23874.

Another preferred acidic alpha-amylase is derived from a strain *Aspergillus niger.* The acid fungal alpha-amylase may be the one from *A. niger* disclosed as "AMYA_ASPNG" in the Swiss-prot/TeEMBL database under the primary accession no. P56271 and described in WO 1989/01969 (Example 3). A commercially available acid fungal alpha-amylase derived from *Aspergillus niger* is SP288 (available from Novozymes A/S, Denmark).

The fungal alpha-amylase may also be a wild-type enzyme comprising a starch-binding domain (SBD) and an alpha-amylase catalytic domain (*i.e.*, none-hybrid), or a variant thereof. In an embodiment, the wild-type alpha-amylase may be derived from a strain of *Aspergillus ka wachii.*

Other contemplated wild-type alpha-amylases include those derived from a strain of the genera *Rhizomucor* and *Meripilus,* preferably a strain of *Rhizomucor pusillus* (WO 2004/055178 or *Meripilus giganteus.*

The alpha-amylase may be derived from *Aspergillus kawachii* as disclosed by Kaneko et al., 1996, J. Ferment. Bioeng. 81:292-298, "Molecular-cloning and determination of the nucleotide-sequence of a gene encoding an acid-stable alpha-amylase from *Aspergillus kawachii";* and further as EMBL:#AB008370.

Fungal Hybrid Alpha-Amylase

One or more fungal hybrid alpha-amylases may be used. The fungal acid alpha-amylase may be a hybrid alpha-amylase. Examples of fungal hybrid alpha-amylases include the ones disclosed in WO 2005/003311 or U.S. Application Publication No. 2005/0054071 (Novozymes) or US patent application no. 60/638,614, see US 2008/0090271 (Novozymes). A hybrid alpha-amylase may comprise an alpha-amylase catalytic domain (CD) and a carbohydrate-binding domain/module (CBM), such as a starch binding domain, and optionally a linker.

Specific examples of contemplated hybrid alpha-amylases include those disclosed in Table 1 to 5 of the examples in US patent application no. 60/638,614, including Fungamyl variant with catalytic domain JA118 and *Athelia rolfsii* SBD (SEQ ID NO:100 in US 60/638,614), *Rhizomucor pusillus* alpha-amylase with *Athelia rolfsii* AMG linker and SBD (SEQ ID NO: 101 in US application no. 60/638,614), *Rhizomucor pusillus* alpha-amylase with *Aspergillus niger* glucoamylase linker and SBD (which is disclosed in Table 5 as a combination of amino acid sequences SEQ ID NO:20, SEQ ID NO:72 and SEQ ID NO:96 in US application no. 11/316,535, see US 2006/0148054 or as V039 in Table 5 in WO 2006/069290, and *Meripilus giganteus* alpha-amylase with *Athelia rolfsii* glucoamylase linker and SBD (SEQ ID NO:102 in US application no. 60/638,614). Other specifically contemplated hybrid alpha-amylases are any of the ones listed in Tables 3, 4, 5, and 6 in Example 4 in US application no. 11/316,535 and WO 2006/069290.

Other specific examples of contemplated hybrid alpha-amylases include those disclosed in U.S. Application Publication no. 2005/0054071, including those disclosed in Table 3 on page 15, such as *Aspergillus niger* alpha-amylase with *Aspergillus kawachii* linker and starch binding domain.

Contemplated are also alpha-amylases which exhibit a high identity to any of above mention alpha-amylases, *i.e.*, more than 70%, more than 75%, more than 80%, more than 85%, more than 90%, more than 95%, more than 96%, more than 97%, more than 98%, more than 99% or even 100% identity to the mature enzyme sequences.

An acid alpha-amylases may according to the invention be added in an amount of 0.1 to 10 AFAU/g DS, preferably 0.10 to 5 AFAU/g DS, especially 0.3 to 2 AFAU/g DS.

Commercial Alpha-Amylase Products

Preferred commercial compositions comprising alpha-amylase include MYCOLASE from DSM, BAN™, TERMAMYL™ SC, FUNGAMYL™, LIQUOZYME™ X and SAN™ SUPER, SAN™ EXTRA L (Novozymes A/S) and CLARASE™ L-40,000, DEX-LO™, SPEZYME™ FRED, SPEZYME™ AA, and SPEZYME™ DELTA AA (Genencor Int.), and the acid fungal alpha-amylase sold under the trade name SP288 (available from Novozymes A/S, Denmark).

Carbohydrate-Source Generating Enzyme

The phrase "carbohydrate-source generating enzyme" includes glucoamylase (being glucose generators), beta-amylase and maltogenic amylase (being maltose generators). A carbohydrate-source generating enzyme is capable of producing a carbohydrate that can be used as an energy-source by the fermenting organism(s) in question, for instance, when used in a process for producing a fermentation product such as ethanol. The generated carbohydrate may be converted directly or indirectly to the desired fermentation product, preferably ethanol. A mixture of carbohydrate-source generating enzymes may be present. Especially contemplated mixtures are mixtures of at least a glucoamylase and an alpha-amylase, especially an acid amylase, even more preferred an acid fungal alpha-amylase.

Glucoamylase

One or more glucoamylases may be used. A glucoamylase may be derived from any suitable source, e.g., derived from a microorganism or a plant. Preferred glucoamylases are of fungal or bacterial origin selected from the group consisting of *Aspergillus* glucoamylases, in particular *A. niger* G1 or G2 glucoamylase (Boel et al., 1984, EMBO J. 3 (5), p. 1097-1102), and variants thereof, such as those disclosed in WO 1992/00381, WO 2000/04136 and WO 2001/04273 (from Novozymes, Denmark); the *A. awamori* glucoamylase disclosed in WO 1984/02921, *A. oryzae* glucoamylase (Agric. Biol. Chem., 1991, 55 (4), p. 941-949), and variants or fragments thereof. Other *Aspergillus* glucoamylase variants include variants with enhanced thermal stability: G137A and G139A (Chen et al., 1996, Prot. Eng. 9, 499-505); D257E and D293E/Q (Chen et al., 1995, Prot. Eng. 8, 575-582); N182 (Chen et al., 1994, Biochem. J. 301, 275-281); disulphide bonds, A246C (Fierobe et al., 1996, Biochemistry, 35, 8698-8704; and introduction of Pro residues in position A435 and S436 (Li et al., 1997, Protein Eng. 10, 1199-1204.

Other glucoamylases include *Athelia rolfsii* (previously denoted *Corticium rolfsii*), glucoamylase (see U.S. Patent No. 4,727,026 and Nagasaka et al., 1998, "Purification and properties of the raw-starch-degrading glucoamylases from Corticium rolfsii, Appl Microbiol Biotechnol 50:323-330), and *Talaromyces* glucoamylases, in particular derived from *Talaromyces emersonii* (WO 1999/28448), *Talaromyces leycettanus* (U.S. Patent No. Re. 32,153), *Talaromyces duponti,* and *Talaromyces thermophilus* (U.S. Patent No. 4,587,215).

Bacterial glucoamylases contemplated include glucoamylases from the genus *Clostridium,* in particular *C. thermoamylolyticum* (EP 135,138) and *C. thermohydrosulfuricum* (WO 1986/01831), and *Trametes cingulata* disclosed in WO 2006/069289.

Hybrid glucoamylases are also contemplated. Examples of the hybrid glucoamylases are disclosed in WO 2005/045018. Specific examples include the hybrid glucoamylase disclosed in Table 1 and 4 of Example 1 of WO 2005/045018.

Contemplated are also glucoamylases that exhibit a high identity to any of the above mentioned glucoamylases, *i.e.*, more than 70%, more than 75%, more than 80%, more than 85% more than 90%, more than 95%, more than 96%, more than 97%, more than 98%, more than 99% or even 100% identity to the mature enzymes sequences.

Commercially available compositions comprising glucoamylase include AMG 200L; AMG 300 L; SAN™ SUPER, SAN™ EXTRA L, SPIRIZYME™ PLUS, SPIRIZYME™ FUEL, SPIRIZYME™ B4U and AMG™ E (from Novozymes A/S); OPTIDEX™ 300 (from Genencor Int.); AMIGASE™ and AMIGASE™ PLUS (from DSM); G-ZYME™ G900, G-ZYME™ and G990 ZR (from Genencor Int.).

Glucoamylases may be added in an amount of 0.02-20 AGU/g DS, preferably 0.1-10 AGU/g DS, especially between 1-5 AGU/g DS, such as 0.5 AGU/g DS.

Beta-amylase

One or more beta-amylases may be used. The term "beta-amylase" (E.C 3.2.1.2) is the name traditionally given to exo-acting maltogenic amylases, which catalyze the hydrolysis of 1,4-alpha-glucosidic linkages in amylose, amylopectin and related glucose polymers. Maltose units are successively removed from the non-reducing chain ends in a step-wise manner until the molecule is degraded or, in the case of amylopectin, until a branch point is reached. The maltose released has the beta anomeric configuration, hence the name beta-amylase.

Beta-amylases have been isolated from various plants and microorganisms (W.M. Fogarty and C.T. Kelly, Progress in Industrial Microbiology, vol. 15, pp. 112-115, 1979). These beta-amylases are characterized by having optimum temperatures in the range from 40°C to 65°C and optimum pH in the range from 4.5 to 7. A commercially available beta-amylase from barley is NOVOZYM™ WBA from Novozymes A/S, Denmark and SPEZYME™ BBA 1500 from Genencor Int., USA.

Maltogenic amylase,

One or more maltogenic amylases may be used. The amylase may also be a maltogenic alpha-amylase. A maltogenic alpha-amylase (glucan 1,4-alpha-maltohydrolase, E.C. 3.2.1.133) is able to hydrolyze amylose and amylopectin to maltose in the alpha-configuration. A maltogenic amylase from *Bacillus stearothermophilus* strain NCIB 11837 is commercially available from Novozymes A/S. Maltogenic alpha-amylases are described in U.S. Patent Nos. 4,598,048, 4,604,355 and 6,162,628. The maltogenic amylase may be added in an amount of 0.05- 5 mg total protein/gram DS or 0.05- 5 MANU/g DS.

Proteases

A protease may be added during hydrolysis, fermentation or simultaneous hydrolysis and fermentation. The protease may be added to deflocculate the fermenting organism, especially yeast, during fermentation. The protease may be any protease. In a preferred embodiment, the protease is an acid protease of microbial origin, preferably of fungal or bacterial origin. An acid fungal protease is preferred, but also other proteases can be used.

Suitable proteases include microbial proteases, such as fungal and bacterial proteases. Preferred proteases are acidic proteases, *i.e.*, proteases characterized by the ability to hydrolyze proteins under acidic conditions below pH 7.

Contemplated acid fungal proteases include fungal proteases derived from *Aspergillus, Mucor, Rhizopus, Candida, Coriolus, Endothia, Enthomophtra, Irpex, Penicillium, Sclerotium* and *Torulopsis.* Especially contemplated are proteases derived from *Aspergillus niger* (see, *e.g*., Koaze et al., 1964, Agr. Biol. Chem. Japan, 28, 216), *Aspergillus saitoi* (see, *e.g*., Yoshida, 1954, J. Agr. Chem. Soc. Japan, 28, 66), *Aspergillus awamori* (Hayashida et al., 1977, Agric. Biol. Chem., 42(5), 927-933, *Aspergillus aculeatus* (WO 1995/02044), or *Aspergillus oryzae,* such as the pepA protease; and acidic proteases from *Mucor pusillus or Mucor miehei.*

Also contemplated are neutral or alkaline proteases, such as a protease derived from a strain of *Bacillus.* For example, protease contemplated for the invention is derived from *Bacillus amyloliquefaciens* and has the sequence obtainable at Swissprot as Accession No. P06832. Also contemplated are the proteases having at least 90% identity to amino acid sequence obtainable at Swissprot as Accession No. P06832 such as at least 92%, at least 95%, at least 96%, at least 97%, at least 98%, or particularly at least 99% identity.

Further contemplated are the proteases having at least 90% identity to amino acid sequence disclosed as SEQ ID NO:1 in WO 2003/048353 such as at 92%, at least 95%, at least 96%, at least 97%, at least 98%, or particularly at least 99% identity.

Also contemplated are papain-like proteases such as proteases within E.C. 3.4.22.* (cysteine protease), such as EC 3.4.22.2 (papain), EC 3.4.22.6 (chymopapain), EC 3.4.22.7 (asclepain), EC 3.4.22.14 (actinidain), EC 3.4.22.15 (cathepsin L), EC 3.4.22.25 (glycyl endopeptidase) and EC 3.4.22.30 (caricain).

In an embodiment, the protease may be a protease preparation derived from a strain of *Aspergillus,* such as *Aspergillus oryzae.* In another embodiment, the protease may be derived from a strain of *Rhizomucor,* preferably *Rhizomucor meihei.* In another contemplated embodiment, the protease may be a protease preparation, preferably a mixture of a proteolytic preparation derived from a strain of *Aspergillus,* such as *Aspergillus oryzae,* and a protease derived from a strain of *Rhizomucor,* preferably *Rhizomucor meihei.*

Aspartic acid proteases are described in, for example, Hand-book of Proteolytic Enzymes, Edited by A.J. Barrett, N.D. Rawlings and J.F. Woessner, Aca-demic Press, San Diego, 1998, Chapter 270). Suitable examples of aspartic acid protease include, *e.g*., those disclosed in R.M. Berka et al., Gene, 96, 313 (1990)); (R.M. Berka et al., Gene, 125, 195-198 (1993)); and Gomi et al., Biosci. Biotech. Biochem. 57, 1095-1100 (1993).

Commercially available products include ALCALASE®, ESPERASE™, FLAVOURZYME™, PROMIX™, NEUTRASE®, RENNILASE®, NOVOZYM™ FM 2.0L, and NOVOZYM™ 50006 (available from Novozymes A/S, Denmark) and GC106™ and SPEZYME™ FAN from Genencor Int., Inc., USA.

The protease may be present in an amount of 0.0001-1 mg enzyme protein per g DS, preferably 0.001 to 0.1 mg enzyme protein per g DS. Alternatively, the protease may be present in an amount of 0.0001 to 1 LAPU/g DS, preferably 0.001 to 0.1 LAPU/g DS and/or 0.0001 to 1 mAU-RH/g DS, preferably 0.001 to 0.1 mAU-RH/g DS.

The present invention is further described by the following examples which should not be construed as limiting the scope of the invention.

Materials and Methods

Identity

The relatedness between two amino acid sequences or between two polynucleotide sequences is described by the parameter "identity".

For purposes of the present invention, the degree of identity between two amino acid sequences is determined by the Clustal method (Higgins, 1989, CABIOS 5: 151-153) using the LASERGENE™ MEGALIGN™ software (DNASTAR, Inc., Madison, WI) with an identity table and the following multiple alignment parameters: Gap penalty of 10 and gap length penalty of 10. Pairwise alignment parameters are Ktuple=1, gap penalty=3, windows=5, and diagonals=5.

For purposes of the present invention, the degree of identity between two polynucleotide sequences is determined by the Wilbur-Lipman method (Wilbur and Lipman, 1983, Proceedings of the National Academy of Science USA 80: 726-730) using the LASERGENE™ MEGALIGN™ software (DNASTAR, Inc., Madison, WI) with an identity table and the following multiple alignment parameters: Gap penalty of 10 and gap length penalty of 10. Pairwise alignment parameters are Ktuple=3, gap penalty=3, and windows=20.

<u>Protease assays</u>

<u>AZCL-casein assay</u>

A solution of 0.2% of the blue substrate AZCL-casein is suspended in Borax/$NaH_2PO_4$ buffer pH9 while stirring. The solution is distributed while stirring to microtiter plate (100 microL to each well), 30 microL enzyme sample is added and the plates are incubated in an Eppendorf Thermomixer for 30 minutes at 45°C and 600 rpm. Denatured enzyme sample (100°C boiling for 20 min) is used as a blank. After incubation the reaction is stopped by transferring the microtiter plate onto ice and the coloured solution is separated from the solid by centrifugation at 3000 rpm for 5 minutes at 4°C. 60 microL of supernatant is transferred to a microtiter plate and the absorbance at 595nm is measured using a BioRad Microplate Reader.

<u>pNA-assay</u>

50 microL protease-containing sample is added to a microtiter plate and the assay is started by adding 100 microL 1 mM pNA substrate (5 mg dissolved in 100 microL DMSO and further diluted to 10 mL with Borax/$NaH_2PO_4$ buffer pH9.0). The increase in $OD_{405}$ at room temperature is monitored as a measure of the protease activity.

Glucoamylase activity (AGU)

Glucoamylase activity may be measured in Glucoamylase Units (AGU).
The Novo Glucoamylase Unit (AGU) is defined as the amount of enzyme, which hydrolyzes 1 micromole maltose per minute under the standard conditions 37°C, pH 4.3, substrate: maltose 23.2 mM, buffer: acetate 0.1 M, reaction time 5 minutes.
An autoanalyzer system may be used. Mutarotase is added to the glucose dehydrogenase reagent so that any alpha-D-glucose present is turned into beta-D-glucose. Glucose dehydrogenase reacts specifically with beta-D-glucose in the reaction mentioned above, forming NADH which is determined using a photometer at 340 nm as a measure of the original glucose concentration.

| <u>AMG incubation:</u> | |
|---|---|
| Substrate: | maltose 23.2 mM |
| Buffer: | acetate 0.1 M |
| pH: | 4.30 ± 0.05 |
| Incubation temperature: | 37°C ± 1 |
| Reaction time: | 5 minutes |
| Enzyme working range: | 0.5-4.0 AGU/mL |

| <u>Color reaction:</u> | |
|---|---|
| GlucDH: | 430 U/L |
| Mutarotase: | 9 U/L |
| NAD: | 0.21 mM |

(continued)

| Color reaction: | |
|---|---|
| Buffer: | phosphate 0.12 M; 0.15 M NaCl |
| pH: | 7.60 ± 0.05 |
| Incubation temperature: | 37°C ± 1 |
| Reaction time: | 5 minutes |
| Wavelength: | 340 nm |

A folder (EB-SM-0131.02/01) describing this analytical method in more detail is available on request from Novozymes A/S, Denmark.

Alpha-amylase activity (KNU)

The alpha-amylase activity may be determined using potato starch as substrate. This method is based on the break-down of modified potato starch by the enzyme, and the reaction is followed by mixing samples of the starch/enzyme solution with an iodine solution. Initially, a blackish-blue color is formed, but during the break-down of the starch the blue color gets weaker and gradually turns into a reddish-brown, which is compared to a colored glass standard.
One Kilo Novo alpha amylase Unit (KNU) is defined as the amount of enzyme which, under standard conditions (i.e., at 37°C +/- 0.05; 0.0003 M $Ca^{2+}$; and pH 5.6) dextrinizes 5260 mg starch dry substance Merck Amylum solubile.
A folder EB-SM-0009.02/01 describing this analytical method in more detail is available upon request to Novozymes A/S, Denmark.

Acid alpha-amylase activity (AFAU)

When used according to the present invention the activity of an acid alpha-amylase may be measured in AFAU (Acid Fungal Alpha-amylase Units). Alternatively, activity of acid alpha-amylase may be measured in AAU (Acid Alpha-amylase Units).

Acid Alpha-amylase Units (AAU)

The acid alpha-amylase activity can be measured in AAU (Acid Alpha-amylase Units), which is an absolute method. One Acid Amylase Unit (AAU) is the quantity of enzyme converting 1 g of starch (100% of dry matter) per hour under standardized conditions into a product having a transmission at 620 nm after reaction with an iodine solution of known strength equal to the one of a color reference.

Standard conditions/reaction conditions:

| | |
|---|---|
| Substrate: | Soluble starch. Concentration approx. 20 g DS/L. |
| Buffer: | Citrate, approx. 0.13 M, pH=4.2 |
| Iodine solution: | 40.176 g potassium iodide + 0.088 g iodine/L |
| City water | 15°-20°dH (German degree hardness) |
| pH: | 4.2 |
| Incubation temperature: | 30°C |
| Reaction time: | 11 minutes |
| Wavelength: | 620 nm |
| Enzyme concentration: | 0.13-0.19 AAU/mL |
| Enzyme working range: | 0.13-0.19 AAU/mL |

The starch should be Lintner starch, which is a thin-boiling starch used in the laboratory as colorimetric indicator. Lintner starch is obtained by dilute hydrochloric acid treatment of native starch so that it retains the ability to color blue with iodine. Further detail can be found in EP 0140,410 B2. Determination of FAU-F

FAU-F Fungal Alpha-Amylase Units (Fungamyl) is measured relative to an enzyme standard of a declared strength.

| Reaction conditions | |
|---|---|
| Temperature | 37°C |
| pH | 7.15 |
| Wavelength | 405 nm |
| Reaction time | 5 min |
| Measuring time | 2 min |

A folder (EB-SM-0216.02) describing this standard method in more detail is available on request from Novozymes A/S, Denmark.

<u>Acid alpha-amylase activity (AFAU)</u>

Acid alpha-amylase activity may be measured in AFAU (Acid Fungal Alpha-amylase Units), which are determined relative to an enzyme standard. 1 AFAU is defined as the amount of enzyme which degrades 5.260 mg starch dry matter per hour under the below mentioned standard conditions.

Acid alpha-amylase, an endo-alpha-amylase (1,4-alpha-D-glucan-glucanohydrolase, E.C. 3.2.1.1) hydrolyzes alpha-1,4-glucosidic bonds in the inner regions of the starch molecule to form dextrins and oligosaccharides with different chain lengths. The intensity of color formed with iodine is directly proportional to the concentration of starch. Amylase activity is determined using reverse colorimetry as a reduction in the concentration of starch under the specified analytical conditions.

$$\text{STARCH + IODINE} \xrightarrow[\text{40°, pH 2,5}]{\text{ALPHA - AMYLASE}} \text{DEXTRINS + OLIGOSACCHARIDES}$$

$\lambda = 590$ nm

blue/violet t = 23 sec. decoloration

<u>Standard conditions/reaction conditions:</u>

| | |
|---|---|
| Substrate: | Soluble starch, approx. 0.17 g/L |
| Buffer: | Citrate, approx. 0.03 M |
| Iodine (I2): | 0.03 g/L |
| $CaCl_2$: | 1.85 mM |
| pH: | 2.50 ± 0.05 |
| Incubation temperature: | 40°C |
| Reaction time: | 23 seconds |
| Wavelength: | 590 nm |
| Enzyme concentration: | 0.025 AFAU/mL |
| Enzyme working range: | 0.01-0.04 AFAU/mL |

A folder EB-SM-0259.02/01 describing this analytical method in more detail is available upon request to Novozymes A/S, Denmark.

<u>Measurement of Cellulase Activity Using Filter Paper Assay (FPU assay)</u>

Source of Method

The method is disclosed in a document entitled "Measurement of Cellulase Activities" by Adney, B. and Baker, J., 1996, Laboratory Analytical Procedure, LAP-006, National Renewable Energy Laboratory (NREL). It is based on the

IUPAC method for measuring cellulase activity (Ghose, T.K., 1987, Measurement of Cellulase Activities, Pure & Appl. Chem. 59: 257-268.

Procedure

The method is carried out as described by Adney and Baker, 1996, *supra,* except for the use of a 96 well plates to read the absorbance values after color development, as described below.

*Enzyme Assay Tubes:*

A rolled filter paper strip (#1 Whatman; 1 X 6 cm; 50 mg) is added to the bottom of a test tube (13 X 100 mm). To the tube is added 1.0 mL of 0.05 M Na-citrate buffer (pH 4.80).
The tubes containing filter paper and buffer are incubated 5 min. at 50°C (± 0.1 °C) in a circulating water bath.
Following incubation, 0.5 mL of enzyme dilution in citrate buffer is added to the tube. Enzyme dilutions are designed to produce values slightly above and below the target value of 2.0 mg glucose.
The tube contents are mixed by gently vortexing for 3 seconds.
After vortexing, the tubes are incubated for 60 mins. at 50°C (± 0.1°C) in a circulating water bath.
Immediately following the 60 min. incubation, the tubes are removed from the water bath, and 3.0 mL of DNS reagent is added to each tube to stop the reaction. The tubes are vortexed 3 seconds to mix.

*2.3 Blank and Controls*

A reagent blank is prepared by adding 1.5 mL of citrate buffer to a test tube.
A substrate control is prepared by placing a rolled filter paper strip into the bottom of a test tube, and adding 1.5 mL of citrate buffer.
Enzyme controls are prepared for each enzyme dilution by mixing 1.0 mL of citrate buffer with 0.5 mL of the appropriate enzyme dilution.
The reagent blank, substrate control, and enzyme controls are assayed in the same manner as the enzyme assay tubes, and done along with them.

*Glucose Standards*

A 100 mL stock solution of glucose (10.0 mg/mL) is prepared, and 5 mL aliquots are frozen. Prior to use, aliquots are thawed and vortexed to mix.
Dilutions of the stock solution are made in citrate buffer as follows:

$$G1 = 1.0\ mL\ stock + 0.5\ mL\ buffer = 6.7\ mg/mL = 3.3\ mg/0.5\ mL$$

$$G2 = 0.75\ mL\ stock + 0.75\ mL\ buffer = 5.0\ mg/mL = 2.5\ mg/0.5\ mL$$

$$G3 = 0.5\ mL\ stock + 1.0\ mL\ buffer = 3.3\ mg/mL = 1.7\ mg/0.5\ mL$$

$$G4 = 0.2\ mL\ stock + 0.8\ mL\ buffer = 2.0\ mg/mL = 1.0\ mg/0.5\ mL$$

Glucose standard tubes are prepared by adding 0.5 mL of each dilution to 1.0 mL of citrate buffer.
The glucose standard tubes are assayed in the same manner as the enzyme assay tubes, and done along with them.

*Color Development*

Following the 60 min. incubation and addition of DNS, the tubes are all boiled together for 5 mins. in a water bath.
After boiling, they are immediately cooled in an ice/water bath.
When cool, the tubes are briefly vortexed, and the pulp is allowed to settle. Then each tube is diluted by adding

50 microL from the tube to 200 microL of $ddH_2O$ in a 96-well plate. Each well is mixed, and the absorbance is read at 540 nm.

*Calculations (examples are given in the NREL document)*

A glucose standard curve is prepared by graphing glucose concentration (mg/0.5 mL) for the four standards (G1-G4) vs. $A_{540}$. This is fitted using a linear regression (Prism Software), and the equation for the line is used to determine the glucose produced for each of the enzyme assay tubes.

A plot of glucose produced (mg/0.5 mL) vs. total enzyme dilution is prepared, with the Y-axis (enzyme dilution) being on a log scale.

A line is drawn between the enzyme dilution that produced just above 2.0 mg glucose and the dilution that produced just below that. From this line, it is determined the enzyme dilution that would have produced exactly 2.0 mg of glucose.

The Filter Paper Units/mL (FPU/mL) are calculated as follows:

$$\text{FPU/mL} = 0.37/\ \text{enzyme dilution producing 2.0 mg glucose.}$$

Example

The effect of adding DDGs on sugar yield was tested. DDGs were added to milled dry corn stover prior to pre-treatment. The sugar content was measured at 24, 48, and 72 hours after the start of hydrolysis.

Cellulase preparation A: Cellulase preparation A is a cellulolytic composition comprising a polypeptide having cellulolytic enhancing activity (GH61A) disclosed in WO 2005/074656; a beta-glucosidase (a fusion protein disclosed in WO 2008/057637); and cellulolytic enzymes preparation derived from Trichoderma reesei. Cellulase preparation A is disclosed in copending international application no. PCT/US2008/065417, see WO 2008/151079.

DDGs were obtained from ADKINS ENERGY LLC (Lena, IL). Cellulase preparation A was used for hydrolysis in an amount of 6 mg enzyme protein/g TS at 5% TS loading. Milled corn stover was mixed with DDGs and slurried in dilute $H_2SO_4$ (1 % on dry solid basis, solid to liquid: 1:10). The slurried mixture was heat treated in a sand bath at 170 °C for 8 minutes. DDGs was added in amounts of 4 wt. % DDGs (based on corn stover) and 16 wt. % DDGs (based on corn stover). The pre-treated corn stover with DDGs was washed before adding Cellulase preparation A. The washed mixture was hydrolyzed by Cellulase preparation A at 50 °C. The content of released sugar was determined by YSI 2700 SELECT method (YSI Life Sciences, Yellow Springs, OH). As shown in Figure 1 and 2, the addition of DDGs during pre-treatment increased the final sugar yield and conversion rate. When 16 wt.% of DDGs was added into corn stover before pre-treatment, the sugar yield increased from 18.6 g/L to 20.8 g/L and the carbohydrate conversion rate improved from 59.7% to 66.8%.

## Claims

**1.** A method for producing a fermentation product from a lignocellulose-containing material, comprising:

(a) combining lignocellulose-containing material and distillers dried grains with solubles (DDG/S);
(b) acid pre-treating the combined lignocallulose-containing material and DDG/S and heat treating at a temperature in the range of 160-220°C for a period of 1-60 minutes;
(c) exposing the acid pre-treated and heat treated combined lignocellulose-containing material and DDG/S to one or more hydrolyzing enzymes; and
(d) fermenting with a fermenting organism to produce a fermentation product, wherein the DDG/S are introduced to the lignocellulose-containing material in an amount of 4-48% w/w DDG/S/lignocellulose-containing material.

**2.** The method of claim 1, wherein the DDG/S are Introduced to the lignocellulose-containing material in an amount of about 16% wlw DDG/S/dry lignocellulose-containing material.

**3.** The method of claim 1 or 2, wherein the lignocellulose-containing material. Is selected from corn stover, corn cobs, corn fiber, switch grass, wheat straw, rice straw, and bagasse.

**4.** A method for enhancing enzymatic hydrolysis of a lignocellulose-containing material, comprising:

(a) introducing an effective lignin-blocking amount of DDG/S to the lignocellulose-containing material prior to acid pre- treatment and heat treatment at a temperature in the range of 160-220°C for a period of 1-60 minutes, and
(b) exposing the acid pre-treated and heat treated lignocellulose-containing material and DDG/S to one or more hydrolyzing enzymes wherein the DDG/S are introduced to the lignocellulose-containing material in an amount of 4-48% wlw DDG/S/lignocellulose-containing material.

**5.** The method of claim 4, wherein the DDG/S are introduced to the lignocellulose-containing material in an amount of about 16% w/w DDG/S/lignocellulose-containing material.

**6.** The method of claim 4 or 5. wherein the lignocelluloso-containing material Is selected from corn stover, corn cobs, corn fiber, switch grass, wheat straw, rice straw, and bagasse.

## Patentansprüche

**1.** Verfahren zum Herstellen eines Fermentationsprodukts aus einem Lignocellulose enthaltendem Material, umfassend:

(a) Kombinieren von Lignocellulose enthaltendem Material und Trockenschlempe (distillers dried grains with solubles; DDG/S);
(b) Säure-Vorbehandeln des kombinierten Lignocellulose enthaltendem Material und DDG/S und Hitzebehandeln bei einer Temperatur in dem Bereich von 160-220 °C für einen Zeitraum von 1-60 Minuten;
(c) Aussetzen des Säure vorbehandelten und hitzebehandelten kombinierten Lignocellulose enthaltenden Materials und DDG/S gegenüber einem oder mehreren hydrolysierenden Enzymen; und
(d) Fermentieren mit einem Fermentationsorganismus, um ein Fermentationsprodukt herzustellen, wobei die DDG/S in das Lignocellulose enthaltende Material in einer Menge von 4-48 % Gew./Gew. DDG/S / Lignocellulose enthaltendes Material eingeführt wird.

**2.** Verfahren nach Anspruch 1, wobei die DDG/S in das Lignocellulose enthaltende Material in einer Menge von etwa 16% Gew./Gew. DDGS / trockenes Lignocellulose enthaltendes Material eingeführt wird.

**3.** Verfahren nach Anspruch 1 oder 2, wobei das Lignocellulose enthaltende Material ausgewählt ist aus Mais ("corn") -Restpflanze, Maiskolben, Maisfaser, Rutenhirse, Weizenstroh, Reisstroh und Bagasse.

**4.** Verfahren zum Verstärken von enzymatischer Hydrolyse eines Lignocellulose enthaltenden Materials, umfassend:

(a) Einführen einer wirksamen Lignin-blockierenden Menge von DDG/S in das Lignocellulose enthaltende Material vor Säure-Vorbehandlung und Hitzebehandlung bei einer Temperatur in dem Bereich von 160-220 °C für einen Zeitraum von 1-60 Minuten, und
(b) Aussetzen des Säure vorbehandelten und hitzebehandelten kombinierten Lignocellulose enthaltenden Materials und DDG/S gegenüber einem oder mehreren hydrolysierenden Enzymen, wobei die DDG/S in das Lignocellulose enthaltende Material in einer Menge von 4-48 % Gew./Gew. DDG/S / Lignocellulose enthaltendes Material eingeführt wird.

**5.** Verfahren nach Anspruch 4, wobei die DDG/S in das Lignocellulose enthaltende Material in einer Menge von etwa 16% Gew./Gew. DDGS / Lignocellulose enthaltendes Material eingeführt wird.

**6.** Verfahren nach Anspruch 4 oder 5, wobei das Lignocellulose enthaltende Material ausgewählt ist aus Mais ("corn") -Restpflanze, Maiskolben, Maisfaser, Rutenhirse, Weizenstroh, Reisstroh und Bagasse.

## Revendications

**1.** Méthode de production d'un produit de fermentation à partir d'une matière contenant de la lignocellulose, comprenant :

(a) la combinaison de la matière contenant de la lignocellulose et de drêches sèches de distillerie avec solubles

(DDG/S) ;
(b) le prétraitement à l'acide de la matière contenant de la lignocellulose et des DDG/S combinées et leur soumission à un traitement thermique à une température dans la plage de 160 à 220°C pendant une période de 1 à 60 minutes ;
(c) l'exposition de la matière contenant de la lignocellulose et des DDG/S combinées prétraitées à l'acide et ayant été soumises à un traitement thermique à une ou plusieurs enzymes hydrolysantes ; et
(d) leur fermentation avec un organisme de fermentation pour produire un produit de fermentation, dans laquelle les DDG/S sont ajoutées à la matière contenant de la lignocellulose en une quantité de 4 à 48 % p/p de DDG/S/matière contenant de la lignocellulose.

**2.** Méthode selon la revendication 1, dans laquelle les DDG/S sont ajoutées à la matière contenant de la lignocellulose en une quantité d'environ 16 % p/p de DDG/S/matière sèche contenant de la lignocellulose.

**3.** Méthode selon la revendication 1 ou 2, dans laquelle la matière contenant de la lignocellulose est choisie parmi la canne de maïs, les rafles de maïs, la fibre de maïs, le panic raide, la paille de blé, la paille de riz, et la bagasse.

**4.** Méthode destinée à améliorer l'hydrolyse enzymatique d'une matière contenant de la lignocellulose, comprenant :

(a) l'ajout d'une quantité de DDG/S efficace pour bloquer la lignine à de la matière contenant de la lignocellulose avant prétraitement à l'acide et traitement thermique à une température dans la plage de 160 à 220°C pendant une période de 1 à 60 minutes, et
(b) l'exposition de la matière contenant de la lignocellulose et des DDG/S combinées prétraitées à l'acide et ayant été soumises à un traitement thermique à une ou plusieurs enzymes hydrolysantes, dans laquelle les DDG/S sont ajoutées à la matière contenant de la lignocellulose en une quantité de 4 à 48 % p/p de DDG/S/matière contenant de la lignocellulose.

**5.** Méthode selon la revendication 4, dans laquelle les DDG/S sont ajoutées à la matière contenant de la lignocellulose en une quantité d'environ 16 % p/p de DDG/S/matière contenant de la lignocellulose.

**6.** Méthode selon la revendication 4 ou 5, dans laquelle la matière contenant de la lignocellulose est choisie parmi la canne de maïs, les rafles de maïs, la fibre de maïs, le panic raide, la paille de blé, la paille de riz, et la bagasse.

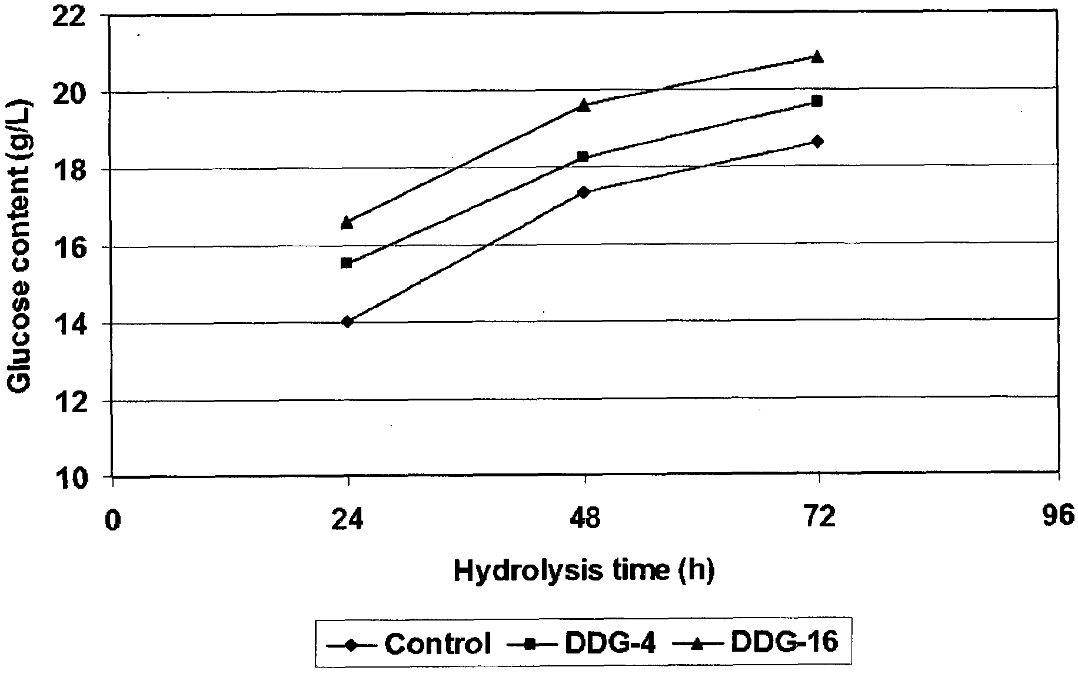
22
20
18
16
14
12
10
Glucose content (g/L)
0
24
48
72
96
Hydrolysis time (h)
Control
DDG-4
DDG-16

Figure 1

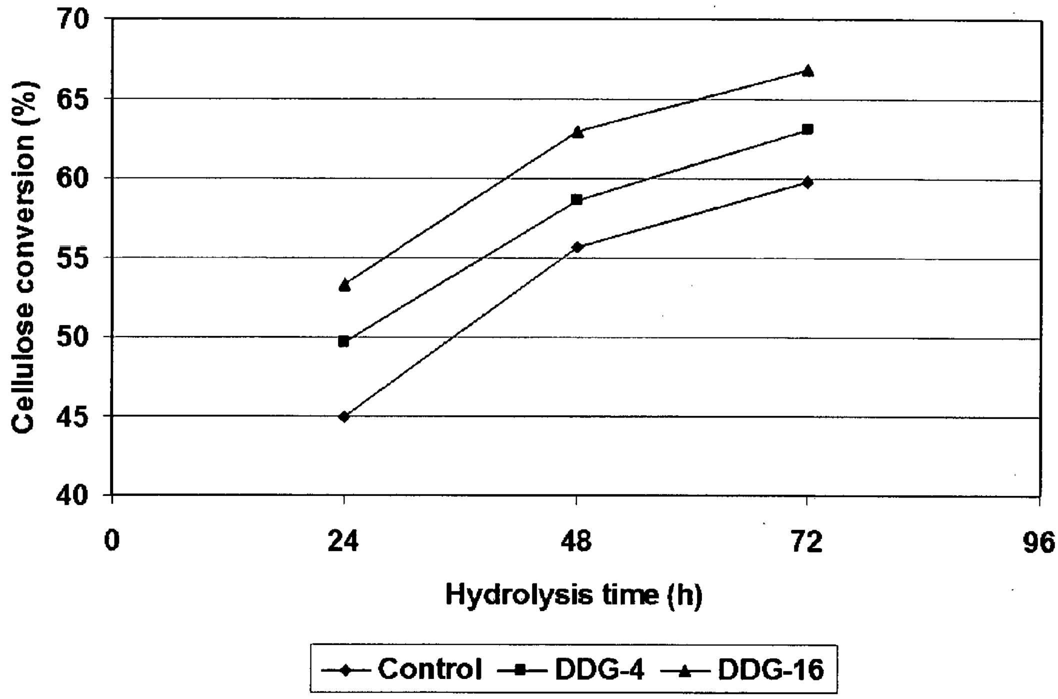

Cellulose conversion (%)
70
65
60
55
50
45
40
0
24
48
72
96
Hydrolysis time (h)
Control
DDG-4
DDG-16


Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2008065417 W
- WO 2005074656 A
- WO 2008057637 A
- EP 562003 A
- WO 2002095014 A
- US 4687742 A
- US 3616221 A
- US 3622463 A
- US 4351903 A
- US 4137126 A
- US 3625828 A
- HU 12415
- DE 2417642
- JP 6928473 B
- WO 2004044129 A
- WO 199919467 A
- WO 199623873 A
- WO 199623874 A
- WO 199741213 A
- WO 200060059 A
- WO 200210355 A
- US 6093562 A
- US 6297038 B
- US 6187576 B
- WO 1996023873 A
- WO 198901969 A
- WO 2004055178 A
- WO 2005003311 A
- US 20050054071 A
- US 60638614 B
- US 20080090271 A
- US 11316535 B
- US 20060148054 A
- WO 2006069290 A
- WO 199200381 A
- WO 200004136 A
- WO 200104273 A
- WO 198402921 A
- US 4727026 A
- WO 199928448 A
- US RE32153 E
- US 4587215 A
- EP 135138 A
- WO 198601831 A
- WO 2006069289 A
- WO 2005045018 A
- US 4598048 A
- US 4604355 A
- US 6162628 A
- WO 199502044 A
- WO 2003048353 A
- EP 0140410 B2
- WO 2008151079 A


### Non-patent literature cited in the description


- **LAU et al.** *Biotechnology and Bioengineering,* 15 February 2008, vol. 99 (3), 529-539
- **PERKINS et al.** *Bioresource Technology,* 2008, vol. 99, 5243-5249
- *Appl. Microbiol. Biotech.,* vol. 77, 61-86
- **HO et al.** *Applied and Environmental Microbiology,* 1998, 1852-1859
- **KARHUMAA et al.** *Microbial Cell Factories,* 2006, vol. 5, 18
- **KUYPER et al.** *FEMS Yeast Research,* 2005, vol. 5, 925-934
- **GHOSE.** *Pure and Appl. Chem.,* vol. 59, 257-268
- **LEVER et al.** *Anal. Biochem.,* 1972, vol. 47, 273-279
- **VAN TILBEURGH et al.** *FEBS Letters,* 1982, vol. 149, 152-156
- **VAN TILBEURGH ; CLAEYSSENS.** *FEBS Letters,* 1985, vol. 187, 283-288
- **VENTURI et al.** *J. Basic Microbiol.,* 2002, vol. 42, 55-66
- *J. Appl.,* 1981, vol. 3, 157-163
- **VIEILLE et al.** *Appl. Environ. Microbiol.,* 1985, vol. 61 (5), 1867-1875
- **VONGSUVANLERT et al.** *Agric. Biol. Chem.,* 1988, vol. 52 (7), 1817-1824
- **OGATA et al.** *Agric. Biol. Chem,* vol. 33, 1519-1520
- **VONGSUVANLERT et al.** *Agric. Biol. Chem,* 1988, vol. 52 (2), 1519-1520
- **KANEKO et al.** *J. Ferment. Bioeng.,* 1996, vol. 81, 292-298
- **BOEL et al.** *EMBO J.,* 1984, vol. 3 (5), 1097-1102
- *Agric. Biol. Chem.,* 1991, vol. 55 (4), 941-949

- **CHEN et al.** *Prot. Eng.,* 1996, vol. 9, 499-505
- **CHEN et al.** *Prot. Eng.,* 1995, vol. 8, 575-582
- **CHEN et al.** *Biochem. J.,* 1994, vol. 301, 275-281
- **FIEROBE et al.** *Biochemistry,* 1996, vol. 35, 8698-8704
- **LI et al.** *Protein Eng.,* 1997, vol. 10, 1199-1204
- **NAGASAKA et al.** Purification and properties of the raw-starch-degrading glucoamylases from Corticium rolfsii. *Appl Microbiol Biotechnol,* 1998, vol. 50, 323-330
- **W.M. FOGARTY ; C.T. KELLY.** *Progress in Industrial Microbiology,* 1979, vol. 15, 112-115
- **KOAZE et al.** *Agr. Biol. Chem. Japan,* 1964, vol. 28, 216
- **YOSHIDA.** *J. Agr. Chem. Soc. Japan,* 1954, vol. 28, 66
- **HAYASHIDA et al.** *Agric. Biol. Chem.,* 1977, vol. 42 (5), 927-933
- Hand-book of Proteolytic Enzymes. Aca-demic Press, 1998
- **R.M. BERKA et al.** *Gene,* 1990, vol. 96, 313
- **R.M. BERKA et al.** *Gene,* 1993, vol. 125, 195-198
- **GOMI et al.** *Biosci. Biotech. Biochem.,* 1993, vol. 57, 1095-1100
- **HIGGINS.** *CABIOS,* 1989, vol. 5, 151-153
- **WILBUR ; LIPMAN.** *Proceedings of the National Academy of Science USA,* 1983, vol. 80, 726-730
- **ADNEY, B. ; BAKER, J.** Measurement of Cellulase Activities. Laboratory Analytical Procedure, LAP-006, 1996
- **GHOSE, T.K.** Measurement of Cellulase Activities. *Pure & Appl. Chem.,* 1987, vol. 59, 257-268